# EUROPEAN PATENT APPLICATION

(11) **EP 2 743 354 A1**
(43) Date of publication of application: **18.06.2014**
(21) Application number: 12197069.3
(22) Date of filing: 13.12.2012
(51) Int. Cl.: C12Q 1/68

(54) **Low melting temperature primer**

(71) Applicant: Alacris Theranostics GmbH, 14195 Berlin (DE)
(72) Inventor: Glökler, Jörn, 10783 Berlin (DE); Mertes, Florian, Dr., 14195 Berlin (DE)
(74) Representative: Fanelli Haag Kilger PLLC

(57) **Abstract**

The present invention relates to a polynucleotide comprising a combination of at least two modifications selected from the group of a d-spacer (abasic site), a short linker, an unlocked nucleic acid, a glycol nucleic acid, a triazole linker and an introduced additional negative charge.

The invention further relates to a method for isothermal amplification of a nucleic acid comprising the steps of (a) combining in a reaction mixture one or more template nucleic acids, an enzyme with amplification activity, at least one polynucleotide comprising a combination of at least one modification selected from the group of a d-spacer (abasic site), a short linker, an unlocked nucleic acid, a glycol nucleic acid, a triazole linker and an introduced additional negative charge, optionally one or more unmodified polynucleotides, a buffer, and deoxynucleotide triphosphates and (b) performing an amplification reaction.

## Description

### Field of the Invention

The present invention is in the field of biology and diagnostics. It is more particularly in the field of molecular biology and in vitro diagnostics. More specifically it is in the field of nucleic acid amplification of preferentially RNA and DNA molecules.

### Background of the Invention

The amplification of nucleic acids has in recent years become a standard method for diagnostics and scientific analysis. However many nucleic acid amplification methods rely on higher temperatures (~72 °C) and utilize enzymes from thermophilic organisms. Many of these reactions can be performed using enzymes from mesophilic organisms. At temperatures below the melting point of the primers however, the sequence specifity of the primers is reduced, resulting in amplification artifacts and unspecific amplifications.

At low temperatures, sites can be targeted for replication by introducing special sequences. These sequences may be targeted by nicking enzymes, or form unusual structures that allow other enzymes to process the given sites. There are no unbiased oligonucleotides that have been modified to hybridize and prime specifically at low temperatures.

Reactions performed with phi29 DNA polymerase at 30°C make use of hexamers. However such hexamers are too short to be specific for distinct targets in a genomic background. Additionally, even hexamers can give rise to artifacts in WGA reactions. One recent approach to avoid artifacts is to irradiate the compounds to render hexamers largely incapable of becoming templates rather than primers (Woyke, T. et al. Decontamination of MDA Reagents for Single Cell Whole Genome Amplification. PLoS ONE 6, e26161 (2011)). However, this may lead to bias against TA rich sequences since T-dimers have less capability of forming stable duplexes with templates for priming.

Hybridisation probes such as molecular beacons employ certain structures such as stems to force strong perfect matching interactions with loop sequences. However, the structure needs to rearrange by the hybridisation event and the stems themselves may interact with non-cognate sequences.

Some DNA modifications are known to decrease annealing temperature. These can be divided under base or backbone modifications. Base modifications that alter the Watson-Crick interaction between opposite bases such as inosine, xanthosine, oxo-guanine, etc. are often biased and can also lead to errors in copying events. Backbone modifications such as acyclic nucleoside analogues lower the melting temperature without affecting the Watson-Crick interaction (Nielsen, P., Dreiøe, L. H. & Wengel, J. Synthesis and evaluation of oligodeoxynucleotides containing acyclic nucleosides: introduction of three novel analogues and a summary. Bioorg. Med. Chem. 3, 19-28 (1995)). Another backbone modification known to decrease annealing temperature can be considered an abasic site (Hianik, T. et al. DNA-duplexes containing abasic sites: correlation between thermostability and acoustic wave properties. Analyst 131, 1161-1166 (2006)) or linker that completely lacks a base to form a pair with the opposite strand.

Another important factor for thermal stability of duplexes is the electrostatic repulsion of the adjacent backbones affected by the negatively charged phosphate groups. Increased stability is observed in hybrids with either absence of any charges such as PNA or additional positive charges introduced by amine groups (Johannsen, M. W., Crispino, L., Wamberg, M. C., Kalra, N. & Wengel, J. Amino acids attached to 2'-amino-LNA: synthesis and excellent duplex stability. Org. Biomol. Chem. 9, 243-252 (2011)).

So far base and nucleoside analogues have been studied to either increase the thermal, chemical or enzymatic stability of duplexes. The increase of specificity of such modifications in longer oligonucleotides for complementary sequences at low temperatures especially in the context of nucleic acid amplification has not been investigated so far.

Surprisingly the inventors found that a combination of multiple backbone modifications does not only lead to a reduction of the melting temperature of polynucleotides, but also leads to a high binding specificity of the oligonucleotide at low temperatures.

### Brief Description of the Invention

The present invention relates to a polynucleotide having a melting temperature of between 15 °C and 65 °C comprising a combination of at least two modifications selected from the group of a d-spacer (abasic site), a short linker, an unlocked nucleic acid, a glycol nucleic acid, a triazole linker and an introduced additional negative charge.

The invention further relates to a method for isothermal amplification of a nucleic acid comprising the steps of (a) combining in a reaction mixture one or more template nucleic acids, an enzyme with amplification activity, at least one polynucleotide comprising at least one modifications selected from the group of a d-spacer (abasic site), a short linker, an unlocked nucleic acid, a glycol nucleic acid, a triazole linker and an introduced additional negative charge, optionally one or more unmodified polynucleotides, a buffer, and deoxynucleotide triphosphates (dNTPs) and (b) performing an isothermal amplification reaction.

Furthermore the invention relates to the use of a polynucleotide having a melting temperature of between 15 °C and 65 °C comprising a combination of at least one or at least two modifications selected from the group of a d-spacer (abasic site), a short linker, an unlocked nucleic acid, a glycol nucleic acid, a triazole linker and an introduced additional negative charge as a primer for the isothermal amplification of a target nucleic acid.

An abasic site may for example be generated by the incorporation of abasic DNA nucleoside analogues, abasic RNA nucleoside analogues and abasic linker molecules that replace the natural backbone structure. A preferred stable abasic DNA nucleoside analogue is based on a 1', 2'-dideoxyribose molecule without a base attached to the C1 atom of the dideoxyribose.

In the context of the present invention a short linker might be an acyclic C3 spacer that provides a similar distance between adjacent bases as a normal nucleotide.

An unlocked nucleic acid (UNA) is an acyclic derivative of RNA lacking the C2'-C3'-bond of the ribose ring of RNA. The acyclic derivative provides greater backbone flexibility without affecting the Watson-Crick pairings.

A glycol nucleic acid (GNA) is a chemical nucleic acid analog, wherein the backbone is in contrast to DNA and RNA not linked by sugar, but by repeating glycol units linked by phosphodiester bonds (Zhang Lilu, Peritz Adam, Meggers Eric (2005). "A simple glycol nucleic acid". J Am Chem Soc 127 (12): 4174-5).

As used herein the term "triazole linker" refers to nucleic acids, wherein the phosphodiester linkage is replaced by a triazole moiety (EI-Sagheer, A. H. et al.;(2011); Biocompatible artificial DNA linker that is read through by DNA polymerases and is functional in Escherischia coli; PNAS; 11338-11343). Triazole linkers can be synthesized chemical ligation of oligonucleotides functionalized with 5'-azide and 3'-alkyne.

As used herein the term modified polynucleotide refers to a polynucleotide comprising at least one backbone modification selected from the group of a d-spacer (abasic site), a short linker, an unlocked nucleic acid, a glycol nucleic acid (GNA) and an introduced additional negative charge. Preferably the polynucleotide comprises a combination of at least two of said modifications.

Non limiting examples for enzymes with amplification activity in the context of this invention are Polymerases, i.e. DNA or RNA polymerases. In a preferred embodiment of the invention the polymerases are working at temperatures between 15 °C to 65 °C. Non-limiting examples for DNA polymerases suitable for use in the context are Bst DNA polymerase, *Thermococcus species* 9°N-7 DNA polymerase, KlenTaq polymerase, Klenow Polymerase and pol Y family polymerases.

As used herein, the term "dNTP" refers to deoxyribonucleoside triphosphates. Non-limiting examples of such dNTPs are dATP, dGTP, dCTP, dTTP, dUTP, which may also be present in the form of labelled derivatives, for instance comprising a fluorescence label, a radioactive label, a biotin label. dNTPs with modified nucleotide bases are also encompassed, wherein the nucleotide bases are for example hypoxanthine, xanthine, 7-methylguanine, inosine, xanthinosine, 7-methylguanosine, 5,6-dihydrouracil, 5-methylcytosine, pseudouridine, dihydrouridine, 5-methylcytidine. Furthermore, ddNTPS of the above-described molecules are encompassed in the present invention.

"Isothermal amplification" refers to amplification which occurs at a single temperature. This does not include the single brief time period (less than 15 minutes) at the initiation of amplification which may be conducted at the same temperature as the amplification procedure or at a higher temperature. Depending on the source of enzymes that are used for the amplification, the reaction can be performed a low temperatures (< 50°C) e.g., at least or about 30, 31, 32, 33, 34, 35, 36, 37, 38, 39, 40, 41, 42, 43, 44, 45, 46, 47, 48, 49 degrees Celsius; or at high temperatures (≥50°C,), e.g., at least or about 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, or more degrees Celsius.

In the context of the present invention, a nucleic acid may be, inter alia, RNA, DNA, cDNA (complementary DNA), LNA (locked nucleic acid), mRNA (messenger RNA), mtRNA (mitochondrial), rRNA (ribosomal RNA), tRNA (transfer RNA), nRNA (nuclear RNA), siRNA (short interfering RNA), snRNA (small nuclear RNA), snoRNA (small nucleolar RNA), scaRNA (Small Cajal Body specific RNA), microRNA, dsRNA (double-stranded RNA), ribozyme, riboswitch, viral RNA, dsDNA (double-stranded DNA), ssDNA (single-stranded DNA), plasmid DNA, cosmid DNA, chromosomal DNA, viral DNA, mtDNA (mitochondrial DNA), nDNA (nuclear DNA), snDNA (small nuclear DNA) or the like or any other class or sub-class of nucleic acid which is distinguishable from the bulk nucleic acid in a sample.

The term "primer" refers to a single stranded nucleic acid capable of binding to a single stranded region on a target nucleic acid to facilitate polymerase dependent replication of the target nucleic acid. Preferably, the primers described herein do not or are not predicted to form secondary structures, complete or partial hairpins, in any given phase of an amplification reaction (e.g., during melting, hybridization/annealing and/or extension).

Generally, primer suitable for use in amplification reactions are short synthetic oligonucleotides, for example, having a length of exactly, about or at least 5, 6, 7, 8, 9, 10, 11, 12, 13, 14, 15, 16, 17, 18, 19, 20, 22, 23, 24, 25, 26, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, or more nucleotide bases. Preferably the primers are between about 5 and 60, 10 and 50, 15 and 30 nucleotide bases.

The term "target" or "template" nucleic acid refers to a whole or part of nucleic acid to be selectively amplified and which is defined by 3' and 5' boundaries. The target nucleic acid may also be referred to as a fragment or sequence that is intended to be amplified. The size of the target nucleic acid to be amplified may be, for example, in the range of about or at least 50 to 1000, 50 to 500, 50 to 250, 75 to 150 bases, kilobases or megabases. The target nucleic acid may be contained within a longer double stranded or single stranded nucleic acid. Alternatively, the target nucleic acid may be an entire double stranded or single stranded nucleic acid. The template can also be modified nucleic acid, e.g. by organic groups such as methyl groups, biotin, formaldehyde modified nucleic acids, and such.

If RNA is used as a template, reverse transcription into cDNA has to be performed prior to amplification. Synthesis of cDNA may be performed prior to amplification in a different reaction and/or different reaction milieu (two-step process) or can be performed within the amplification reagents (one-step process). The target nucleic acid may be damaged and repaired prior amplification (e.g. repair of abasic sites). The target nucleic acid may have no primer binding site. In this case the missing primer binding site may be attached e.g. by ligation so that amplification can be performed.

As used herein the term "lomer" is used synonymously for polypeptide comprising at least one modification or a combination of at least two modifications selected from the group of a d-spacer (abasic site), a short linker, an unlocked nucleic acid, a glycol nucleic acid and an introduced additional negative charge.

As used herein, a kit is a packaged combination optionally including instructions for use of the combination and/or other reactions and components for such use.

### Detailed Description of the Invention

The invention relates to polynucleotides, which are suitable as primers in amplification reactions, which are performed at low temperatures. Hence the invention in particular relates to a polynucleotide having a melting temperature of between 15 °C and 65 °C comprising a combination of at least two modifications selected from the group of a d-spacer (abasic site), a short linker, an unlocked nucleic acid (UNA), a glycol nucleic acid (GNA), a triazole linker and an introduced additional negative charge.

The Inventors found unexpectedly, that the introduction of a combination of backbone modifications into a polynucleotide primer, leads to a decreased melting temperature of the polynucleotide, while still retaining a high specificity for its target region.

Since the polynucleotide is to be used as a primer in low temperature isothermal amplification reactions, it should not be too long. Hence the invention relates to a polynucleotide comprising a combination of at least two modifications selected from the group of a d-spacer (abasic site), a short linker, an unlocked nucleic acid, a glycol nucleic acid, a triazole linker and an introduced additional negative charge, with a length of less than 100 nucleotides, preferably less than 50 nucleotides, more preferably less than 30 nucleotides, more preferably less than 20 nucleotides. Preferably the nucleotide is larger than or exactly four nucleotides long.

In a particular embodiment of the Invention said polynucleotide is a hexamer.

The modifications of the polynucleotide should be located in the backbone and should preferably not affect the Watson-Crick interactions of the nucleotides. Furthermore the polynucleotides must still be acceptable primers for enzymes with amplification activity. Usually copying of moderately modified oligonucleotides can be achieved with special polymerases, but is usually limited by the amount of modifications in a template. UNA can be copied using Therminator DNA polymerase (*Thermococcus species* 9°N-7 DNA polymerase) and to a lesser extent by other polymerases (Dubois, C., Campbell, M. A., Edwards, S. L., Wengel, J. & Veedu, R. N. Stepping towards highly flexible aptamers: enzymatic recognition studies of unlocked nucleic acid nucleotides. Chemical Communications 48, 5503 (2012)). GNA can be extended using Bst DNA polymerase or Therminator DNA polymerase (Tsai, C.-H., Chen, J. & Szostak, J. W. Enzymatic synthesis of DNA on glycerol nucleic acid templates without stable duplex formation between product and template. Proc. Natl. Acad. Sci. U.S.A. 104, 14598-14603 (2007)). Abasic sites can also be bypassed by some polymerases such as the KlenTaq following the A-rule (Obeid, S., Welte, W., Diederichs, K. & Marx, A. Amino Acid Templating Mechanisms in Selection of Nucleotides Opposite Abasic Sites by a Family A DNA Polymerase. J. Biol. Chem. 287, 14099-14108 (2012)). Short linkers such as trimethylene and even 12-methylene have been shown to be bypassed both in vivo and in vitro by a pol Y family polymerase (Maor-Shoshani, A., Ben-Ari, V. & Livneh, Z. Lesion bypass DNA polymerases replicate across non-DNA segments. PNAS 100, 14760-14765 (2003)). However, flexible linkers may allow looping in the lesion bypass thus leading to deletions in the copied sequence.

Further modifications, which affect the melting temperature of nucleic acid oligonucleotides are known to those skilled in the art (Freier, S. and Altmann, K.-H.; The ups and downs of nucleic acid stability: structure-stability studies on chemically-modified DNA:RNA duplexes; Nucleic Acids Research 25 (1997); 4429-4443).

In a preferred embodiment of the invention the backbone modifications of the polynucleotide are at internal positions.

In one embodiment of the invention the backbone modification is an additional negative charge in the backbone.

In a preferred embodiment of the invention the additional negative charge is a linker to the sugar side of the base, a ribose modification or a phosphate analogue.

In a certain embodiment of the invention the polynucleotide contains at least one unmodified nucleotide between two backbone modifications.

In another embodiment of the invention two or more backbone modifications are present side by side, e.g. a chain of unlocked nucleic acids.

In a particular embodiment of the invention the polynucleotide allows a specific binding to at least 6 bases, preferably at least 15 bases, more preferably at least 20 bases of the target nucleic acid.

The backbone modifications of the polynucleotide comprising a combination of at least two modifications selected from the group of a d-spacer (abasic site), a short linker, an unlocked nucleic acid, a glycol nucleic acid, a triazole linker and an introduced additional negative charge might be of the same type or of different types. Hence in one embodiment of the invention, of the at least two modifications two are of the same type. In another embodiment of the invention, of the at least two modifications at least one modification is of a different type then the others.

Beside the length of the polynucleotide there is no limit to the number of backbone modifications, which might be incorporated into the polypeptide.

Since the polynucleotide is preferably intended to be used as a primer in isothermal amplification reactions the combination of backbone modifications has to be selected in way that the polynucleotide can be amplified by enzymes with amplification activity, in particular DNA polymerases. Non limiting examples for Polymerases to amplify the polynucleotide are: Bst DNA polymerase, *Thermococcus species* 9°N-7 DNA polymerase, KlenTaq polymerase, Klenow polymerase and pol Y family polymerases.

The polynucleotide might comprise additional modifications besides the backbone modifications depending on its intended use. In one embodiment of the invention these additional modifications are at the 3' and/or 5' end of the polynucleotide. In another embodiment of the invention additional modifications might be present at internal positions.

In a particular embodiment of the invention the polynucleotide comprises labels as additional modifications. In a preferred embodiment of the invention the labels are fluorescence or quencher labels.

In a preferred embodiment the optional label is a fluorophore, preferably selected from the group of fluorophores comprising 5 or 6 carboxyfluorescein (FAMT™), VIC™, NED™, fluorescein, fluorescein isothiocyanate (FITC), IRD-700/800, cyanine dyes, such as CY3™ , CY5™, CY3.5™, CY5.5™, Cy7™, xanthen, 6-carboxy-2',4',7',4,7-hexachlorofluorescein (HEX), 6-carboxy-1,4-dichloro-2',7'-dichloro-fluorescein (TET^{®}), 6-carboxy-4',5'-dichloro-2',7'-dimethodyfluorescein (JOE™), N,N,N',N'-tetramethyl-6-carboxyrhodamine (TAMRA™), 6-carboxy-X-rhodamine (ROX), 5-carboxyrhodamine-6G (R6G5), 6-carboxyrhodamine-6G (RG6), rhodamine, rhodamine green, rhodamine red, rhodamine 110, Rhodamin 6G^{®}, BODIPY dyes, such as BODIPY TMR, oregon green, coumarines, such as umbelliferone, benzimides, such as Hoechst 33258; phenanthridines, such as Texas Red^{®}, California Red^{®}, Yakima Yellow, Alexa Fluor^{®} 350, Alexa Fluor^{®} 405, Alexa Fluor^{®} 430, Alexa Fluor^{®} 488, Alexa Fluor^{®} 500, Alexa Fluor^{®} 514, Alexa Fluor^{®}532, Alexa Fluor^{®} 546, Alexa Fluor^{®} 555, Alexa Fluor^{®} 568, Alexa Fluor^{®} 594, Alexa Fluor^{®} 610, Alexa Fluor^{®} 633, Alexa Fluor^{®} 647, Alexa Fluor^{®} 660, Alexa Fluor^{®} 680, Alexa Fluor^{®} 700, Alexa Fluor^{®} 750, PET^{®}, ethidium bromide, acridinium dyes, carbazol dyes, phenoxazine dyes, porphyrine dyes, polymethin dyes, Atto 390, Atto 425, Atto 465, Atto 488, Atto 495, Atto 520, Atto 532, Atto 550, Atto 565, Atto 590, Atto 594, Atto 620, Atto 633, Atto 647N, Atto 655, Atto RhoG6, Atto Rho11, Atto Rho12, Atto Rho101, BMN™-5, BMN™-6, CEQ8000 D2, CEQ8000 D3, CEQ8000 D4, DY 480XL, DY 485XL, DY-495, DY-505, DY-510XL, DY-521XL, DY-521XL, DY-530, DY-547, DY-550, DY-555, DY-610, DY-615, DY-630, DY-631, DY-633, DY-635, DY-647, DY-651, DY-675, DY-676, DY-680, DY-681, DY-700, DY-701, DY-730, DY-731, DY-732, DY-750, DY-751, DY-776, DY-780, DY-781, DY-782, 6 carboxy-4',5'-dichloro-2',7'-dimethoxy-fluorescein (JOE), TET™, CAL Fluor^{®} Gold 540, CAL Fluor RED 590, CAL Fluor Red 610, CAL Fluor Red 635, IRDye^{®} 700Dx, IRDye^{®} 800CW, Marina Blue^{®}, Pacific Blue^{®}, Yakima Yellow^{®}, 6-(4,7-Dichloro-2',7'-diphenyl-3',6'-dipivaloylfluorescein-6-carboxamido)-hexyl-1-O-(2-cyano-ethyl)-(N,N-diiso-propyl)-phospho-ramidite (SIMA), CAL Fluor^{®} Gold 540, CAL Fluor^{®} Orange 560, CAL Fluor Red 635, Quasar 570, Quasar 670, LIZ, Sunnyvale Red, LC Red^{®} 610, LC Red^{®} 640, LC Red^{®}670, and LC Red^{®} 705. In a further preferred embodiment of the present invention the label is selected from the group of fluorophores consisting of Atto 465, DY-485XL, FAM™, Alexa Fluor^{®} 488, DY-495, Atto 495, DY-510XL, JOE, TET™, CAL Fluor^{®} Gold 540, DY-521XL, Rhodamin 6G^{®}, Yakima Yellow^{®}, Atto 532, Alexa Fluor^{®}532, HEX, SIMA, Atto RhoG6, VIC, CAL Fluor Orange 560, DY-530, TAMRA™, Quasar 570, Cy3™, NED™, DY-550, Atto 550, Alexa Fluor^{®} 555, PET^{®}, CAL Fluor RED 590, ROX, Texas Red^{®}, CAL Fluor Red 610, CAL Fluor Red 635, Atto 633, Alexa Fluor^{®} 633, DY-630, DY-633 ,DY-631, LIZ, Quasar 670, DY-635, and Cy5™. In a yet further preferred embodiment the label is selected from group of fluorophores consisting of FAM™, DY-510XL, DY-530, and Atto 550.

"Quenching" refers to any process which decreases the fluorescence intensity of a given substance. "Quencher" in context of the present invention relates to a residue which is suited for quenching, e.g. the basic fluorescence of the label without activation. A variety of processes can result in quenching, such as excited state reactions, energy transfer, complex-formation and collisional quenching. As a consequence, quenching is often heavily dependent on pressure and temperature. Molecular oxygen and the iodide ion are common chemical quenchers. Quenching poses a problem for non-instant spectroscopic methods, such as laser-induced fluorescence. Quenching is the basis for Fluorescence Resonance Energy Transfer (FRET) assays. Quenching and dequenching upon interaction with a specific molecular biological target is the basis for activatable optical contrast agents for molecular imaging. There are a few distinct mechanisms by which energy can be transferred: non-radiatively (without absorption or emission of photons) between two dyes, a donor and an acceptor. Förster resonance energy transfer, Dexter energy transfer, Exciplex (excited state complex) formation, and static quenching (also referred to as contact quenching). The skilled artisan is able to choose quenchers according to the needs.

In another embodiment of the invention said polypeptide comprises a free and/or phosphorylated 3' and/or 5' end.

The Invention further relates to a method for isothermal amplification of a nucleic acid comprising the steps of: a)combining in a reaction mixture one or more template nucleic acids, an enzyme with isothermal amplification activity, at least one polynucleotide comprising at least one backbone modification selected from the group comprising a d-spacer (abasic site), a short linker, an unlocked nucleic acid, a glycol nucleic, a triazole linker acid and an introduced additional negative charge as a primer, optionally one or more unmodified polynucleotides, a buffer, and deoxynucleotide triphosphates (dNTPs) and b) performing an amplification reaction.

In a preferred embodiment the invention relates to a method for isothermal amplification of a nucleic acid, wherein the polynucleotide comprising at least one preferably at least two backbone modification selected from the group comprising a d-spacer (abasic site), a short linker, an unlocked nucleic acid, a glycol nucleic acid, a triazole linker and an introduced additional negative charge is a polynucleotide as described above.

Non limiting examples of amplification reactions according to the present invention are: whole genome amplification (WGA), random primer whole genome amplification, rolling circle amplification (RCA), loop-mediated isothermal amplification (LAMP), helicase dependent amplification (HDA) or a multiple displacement amplification (MDA).

Isothermal amplification reactions can be performed at different temperatures. In one embodiment of the invention the isothermal amplification reaction is performed at a temperature below 65 °C, preferably at a temperature between 15 and 65 °C, more preferably between 15 and 50 °C, even more preferably between 15 and 40 °C.

The inventors found, that the enzyme with amplification activity has to be chosen depending on the modifications in the polynucleotide. Non-limiting examples for enzymes suitable for amplification of modified polynucleotides are Bst DNA polymerase, *Thermococcus species* 9°N-7 DNA polymerase, KlenTaq polymerase, Klenow polymerase and pol Y family polymerases.

The inventors found, that the modified polynucleotides allow a specific binding at low temperatures. Where normal primer have high melting temperatures and provide a lower binding specifity at lower temperatures, the modified polynucleotide primers allow a specific binding with a reduced background. Hence in one embodiment of the invention relates to method of isothermal amplification of a nucleic acid, wherein a polynucleotide comprising at least one or two backbone modifications is used as a specific primer and is binding specifically to at least 20 bases, preferably specifically to at least 15 bases, more preferably to at least 10 bases, most preferably to at least 6 bases.

The Inventors further found, that the method is not limited to specific amplification reactions but also for random primer applications, like random primer whole genome amplification. Hence the invention further relates to a method for isothermal amplification of a nucleic acid, wherin wherein a polynucleotide comprising at least one or two backbone modifications is a random primer.

The invention further relates to a method for isothermal amplification of a nucleic acid, wherin wherein a polynucleotide comprising at least one or two backbone modifications has a length of less than 100 nuleotides, preferably less than 50 nucleotides, more preferably less than 30 nucleotides, even more preferably less than 20 nucleotides. Preferably the nucleotide is larger than or exactly four nucleotides long.

In a particular embodiment the invention relates to a method for isothermal amplification of a nucleic acid, wherein a polynucleotide comprising at least one or two backbone modifications is a hexamer.

The inventors found also that the amplification reaction is not limited to singleplex amplification reactions. Hence in one embodiment the invention relates to a method for isothermal amplification of a nucleic acid, wherein the amplification reaction is a singleplex amplification.

In another embodiment the invention relates to a method for isothermal amplification of a nucleic acid, wherein the amplification reaction is a multiplex amplification.

Preferably the amplification product or products are detected or sequenced.

In one embodiment of the invention the amplification product may for example be used for sequencing or next generation sequencing.

DNA sequencing techniques are of major importance in a wide variety of fields ranging from basic research to clinical diagnosis. The results available from such technologies can include information of varying degrees of specificity. For example, useful information can consist of determining whether a particular polynucleotide differs in sequence from a reference polynucleotide, confirming the presence of a particular polynucleotide sequence in a sample, determining partial sequence information such as the identity of one or more nucleotides within a polynucleotide, determining the identity and order of nucleotides within a polynucleotide, etc.

The sequencing step is preferably done by means of next generation sequencing. Manufacturers and technologies are Solexa/Illumina which generate up to 600 Gigabase (Gb) of 36 or 150 bp, Roche/454 which generate up to 700 Mbp reads of 400-1000 bp, ABI/SOLiDTM which generate > 20 Gb/day reads of 35-75 bp, Helicos which generate 21 - 35 Gb reads of 25-45 bp and Complete Genomics (a service company). Other manufacturers include Pacific Bioscience commercializing PacBio RS.

The Solexa/Illumina sequencing by synthesis technology is based on reversible dye-terminators. DNA molecules are first attached to primers on a slide and amplified so that local clonal colonies are formed (bridge amplification). Four types of reversible terminator bases (RT-bases) are added, and non-incorporated nucleotides are washed away. Unlike pyrosequencing, the DNA can only be extended one nucleotide at a time. A camera takes images of the fluorescently labelled nucleotides, then the dye along with the terminal 3' blocker is chemically removed from the DNA, allowing the next cycle (Brenner et al., Nature Biotechnol. 2000. 18(6):630-634).

The SOLiD^{™} ("Sequencing by Oligonucleotide Ligation and Detection") method (Life Technologies; WO 06/084132 A2) is based on the attachment of PCR amplified fragments of template nucleic acids via universal adapter sequences to magnetic beads and subsequent detection of the fragment sequences via ligation of labelled probes to primers hybridized to the adapter sequences. For the read-out a set of four fluorescently labelled di-base probes are used. After read-out, parts of the probes are cleaved and new cycles of ligation, detection and cleavage are performed. Due to the use of di-base probes, two rounds of sequencing have to be performed for each template sequence. Other methods and devices include Ion Torrent and Roche's 454.

PacBio RS is a single molecule real-time sequencing (SMRT) platform based on the properties of zero-mode waveguides (ZMW). A single DNA polymerase enzyme is affixed at the bottom of a ZMW with a single molecule of DNA as a template. The ZMW is a structure that creates an illuminated observation volume that is small enough to observe only a single nucleotide of DNA being incorporated by DNA polymerase. Each of the four DNA nucleotides is attached to one of four different fluorescent dyes. When a nucleotide is incorporated by the DNA polymerase, the fluorescent tag is cleaved off and diffuses out of the observation area of the ZMW where its fluorescence is no longer observable. A detector detects the fluorescent signal of the nucleotide incorporation, and the base call is made according to the corresponding fluorescence of the dye.

GridION and MinION systems developed by Oxford Nanopore Technologies use biological nanopores to sequence single molecules in a so-called DNA strand sequencing approach. A mutant variant of the pore protein, alpha-hemolysin, is inserted in a synthetic proprietary polymer bilayer that restricts ion passage to the pores alone. ONT uses a special enzyme to process the DNA, not the DNA polymerases currently used by some academic teams. The enzyme is capable of ratcheting DNA through the pore at upwards of 1,000 bases/second, a rate that is controlled by various cofactors in solution. The sequence is read by monitoring an electrical signal as a strand of DNA passes through the pore protein. An informatics system reads overlapping nucleotide triplets in the middle of the pore to deduce the sequence. By engineering a hairpin at the end of the molecule, both strands of the template DNA can be sequenced in succession on the same pore. This redundancy is used to reduce the error rate to an acclaimed 4%. Also these sequencing methods are claimed in the method herein.

The current method has the advantage that it is not restricted to a particular sequencing method. If the sequencing step is done by next generation sequencing, it is preferred that the method applied is selected from the group of those described above.

The amplification product may additionally be detected and/or quantified.

The detection step may be done by incorporating into the amplification product detectable probes, e.g. fluorescently labelled probes. A probe according to the present invention is an oligonucleotide, nucleic acid or a fragment thereof, which is substantially complementary to a specific nucleic acid sequence. Suitable hybridization probes include the LightCycler probe (Roche), the TaqMan probe (Life Technologies), a molecular beacon probe, a Scorpion primer, a Sunrise primer, a LUX primer and an Amplifluor primer.

The detection step may be alternatively done by using double-stranded DNA-binding dyes (e.g. SYBR Green) as reporters in a real-time PCR. A DNA-binding dye binds to all double-stranded DNA in PCR, causing fluorescence of the dye. An increase in DNA product during PCR therefore leads to an increase in fluorescence intensity and is measured at each cycle, thus allowing DNA concentrations to be quantified.

The quantification step may be based on quantitative real-time PCR using the techniques described before.

In a particular embodiment the invention relates to a method for isothermal amplification of a nucleic acid, wherein the amplicon is identified by hybridization with a labeled probe, by the accumulation of pyrophosphate, by quenching of 2-aminopurine or other labeled bases in the oligonucleotide primers, by DNA binding dyes, e.g. SybrGreen or ethidium bromide or by intercalating labels for abasic sites in duplex DNA.

If the amplicon is identified by hybridization with a labeled probe, the label might be a fluorescence label or a quencher label.

In a preferred embodiment the invention relates to a method for isothermal amplification of a nucleic acid, wherein the modified polynucleotide comprises a quencher label, which is compatible with the fluorescence label of the probe. Alternatively the modified polynucleotide comprises a fluorescence label and the probe comprises a compatible quencher label.

In one embodiment of the invention the fluorescence label of the probe and/or the modified polynucleotide is a fluorophore, preferably selected from the group of fluorophores comprising 5 or 6 carboxyfluorescein (FAM™), VIC™, NED™, fluorescein, fluorescein isothiocyanate (FITC), IRD-700/800, cyanine dyes, such as CY3™, CY5™, CY3.5™, CY5.5™, Cy7™, xanthen, 6-carboxy-2',4',7',4,7-hexachlorofluorescein (HEX), 6-carboxy-1,4-dichloro-2',7'-dichloro-fluorescein (TET^{®}), 6-carboxy-4',5'-dichloro-2',7'-dimethodyfluorescein (JOE™), N,N,N',N'-tetramethyl-6-carboxyrhodamine (TAMRA™), 6-carboxy-X-rhodamine (ROX), 5-carboxyrhodamine-6G (R6G5), 6-carboxyrhodamine-6G (RG6), rhodamine, rhodamine green, rhodamine red, rhodamine 110, Rhodamin 6G^{®}, BODIPY dyes, such as BODIPY TMR, oregon green, coumarines, such as umbelliferone, benzimides, such as Hoechst 33258; phenanthridines, such as Texas Red^{®}, California Red^{®}, Yakima Yellow, Alexa Fluor^{®} 350, Alexa Fluor^{®} 405, Alexa Fluor^{®} 430, Alexa Fluor^{®} 488, Alexa Fluor^{®} 500, Alexa Fluor^{®} 514, Alexa Fluor^{®}532, Alexa Fluor^{®} 546, Alexa Fluor^{®} 555, Alexa Fluor^{®} 568, Alexa Fluor^{®} 594, Alexa Fluor^{®} 610, Alexa Fluor^{®} 633, Alexa Fluor^{®} 647, Alexa Fluor^{®} 660, Alexa Fluor^{®} 680, Alexa Fluor^{®} 700, Alexa Fluor^{®} 750, PET^{®}, ethidium bromide, acridinium dyes, carbazol dyes, phenoxazine dyes, porphyrine dyes, polymethin dyes, Atto 390, Atto 425, Atto 465, Atto 488, Atto 495, Atto 520, Atto 532, Atto 550, Atto 565, Atto 590, Atto 594, Atto 620, Atto 633, Atto 647N, Atto 655, Atto RhoG6, Atto Rho11, Atto Rho12, Atto Rho101, BMN™-5, BMN™-6, CEQ8000 D2, CEQ8000 D3, CEQ8000 D4, DY 480XL, DY 485XL, DY-495, DY-505, DY-510XL, DY-521XL, DY-521XL, DY-530, DY-547, DY-550, DY-555, DY-610, DY-615, DY-630, DY-631, DY-633, DY-635, DY-647, DY-651, DY-675, DY-676, DY-680, DY-681, DY-700, DY-701, DY-730, DY-731, DY-732, DY-750, DY-751, DY-776, DY-780, DY-781, DY-782, 6 carboxy-4',5'-dichloro-2',7'-dimethoxy-fluorescein (JOE), TET™, CAL Fluor^{®} Gold 540, CAL Fluor RED 590, CAL Fluor Red 610, CAL Fluor Red 635, IRDye^{®} 700Dx, IRDye^{®} 800CW, Marina Blue^{®}, Pacific Blue^{®}, Yakima Yellow^{®}, 6-(4,7-Dichloro-2',7'-diphenyl-3',6'-dipivaloylfluorescein-6-carboxamido)-hexyl-1-O-(2-cyano-ethyl)-(N,N-diiso-propyl)-phospho-ramidite (SIMA), CAL Fluor^{®} Gold 540, CAL Fluor^{®} Orange 560, CAL Fluor Red 635, Quasar 570, Quasar 670, LIZ, Sunnyvale Red, LC Red^{®} 610, LC Red^{®} 640, LC Red^{®}670, and LC Red^{®} 705. In a further preferred embodiment of the present invention the label is selected from the group of fluorophores consisting of Atto 465, DY-485XL, FAM™, Alexa Fluor^{®} 488, DY-495, Atto 495, DY-510XL, JOE, TET™, CAL Fluor^{®} Gold 540, DY-521XL, Rhodamin 6G^{®}, Yakima Yellow^{®}, Atto 532, Alexa Fluor^{®}532, HEX, SIMA, Atto RhoG6, VIC, CAL Fluor Orange 560, DY-530, TAMRA™, Quasar 570, Cy3™, NED™, DY-550, Atto 550, Alexa Fluor^{®} 555, PET^{®}, CAL Fluor RED 590, ROX, Texas Red^{®}, CAL Fluor Red 610, CAL Fluor Red 635, Atto 633, Alexa Fluor^{®} 633, DY-630, DY-633 ,DY-631, LIZ, Quasar 670, DY-635, and Cy5™. In a yet further preferred embodiment the label is selected from group of fluorophores consisting of FAM™, DY-510XL, DY-530, and Atto 550.

Additionally the invention relates to the use of a polynucleotide comprising at least one modifications selected from the group of a d-spacer (abasic site), a short linker, an unlocked nucleic acid, a glycol nucleic acid and an introduced additional negative charge as a primer for the isothermal amplification of a target nucleic acid.

In a preferred the invention relates to the use of a polynucleotide as described herein comprising a combination of at least one modifications selected from the group of a d-spacer (abasic site), a short linker, an unlocked nucleic acid, a glycol nucleic acid and an introduced additional negative charge as a primer for the isothermal amplification of a target nucleic acid.

The invention further relates to a kit comprising at least one polynucleotide as described herein comprising at least one or two modifications selected from the group of a d-spacer (abasic site), a short linker, an unlocked nucleic acid, a glycol nucleic acid and an introduced additional negative charge.

In a preferred embodiment of the invention the kit additionally comprises an enzyme with amplification activity and optionally a buffer and/or deoxyoligonucleotide triphosphates.

In a more preferred embodiment of the invention the kit further comprises at least one labeled probe for the detection of the amplicon.

### Examples

### Example 1: Priming assay

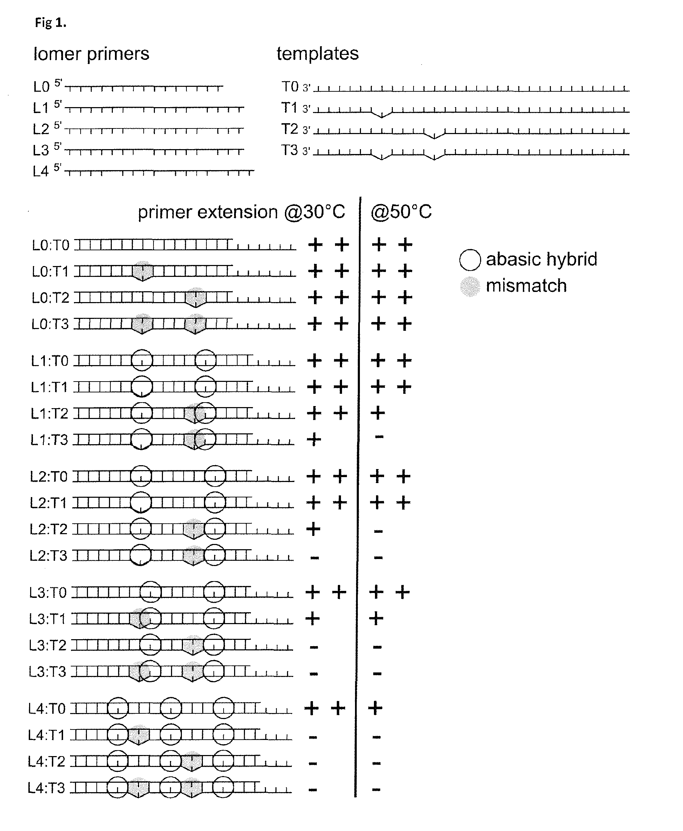

Klenow exo- DNA polymerase mediated extension was performed with equimolar combinations of template DNA and lomer primers. DNA templates consisted of 58 mer synthesized DNA fragments containing single and/or double mismatches within the corresponding Lomer primer binding site. lomer primers consisted of 17 and 18 mer synthesized DNA fragments with two or three interspersed abasic sites. Synthesized DNA sequences are listed in table 1. A general schematic of the reactions is shown in figure 1.

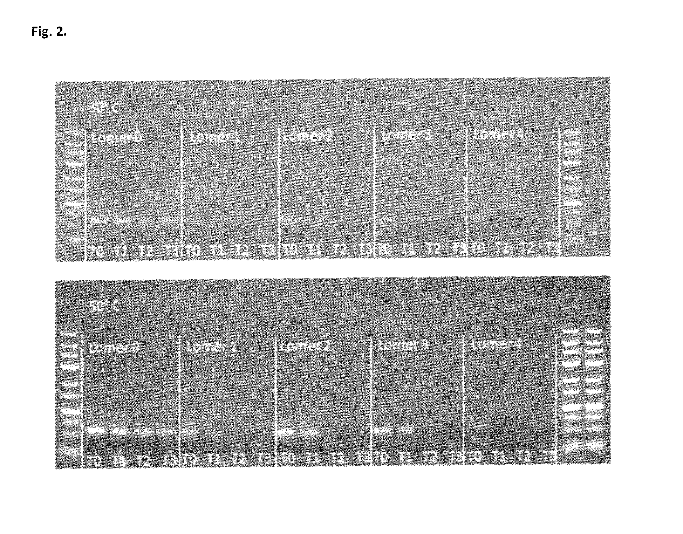

Klenow exo- DNA polymerase reactions were carried out in 10 µL reaction volumes according to the following protocol: 1 µL 10x Klenow exo- DNA polymerase buffer, 1 unit Klenow exo- DNA polymerase, 1 µM template DNA, 1 µM Lomer primer and 0.4 mM dNTPs. Reaction mixtures were prepared on ice with subsequent transfer to the matching reaction temperature of either 30° C or 50° C. for 30 minutes. Visualization of reaction products was performed by agarose gel electrophoresis (figure 2).

### Example 2: Whole Genome Amplification

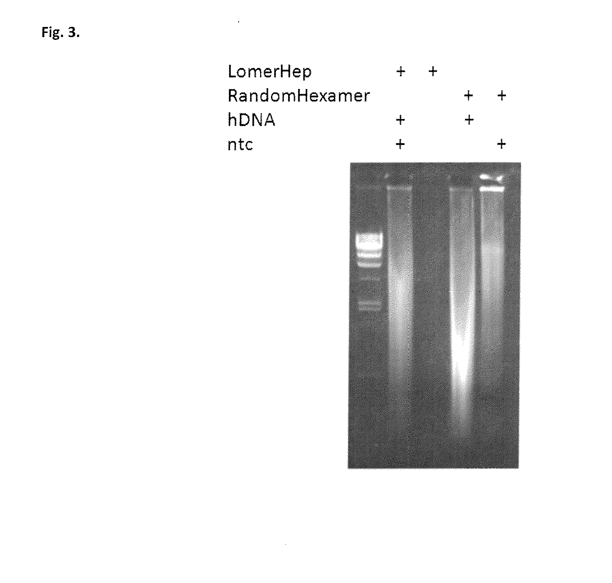

Phi29 DNA polymerase was incubated with either heptamer lomers with one abasic site and phosphororothioate modifications (NNNdNN*N*N format) or with conventional random hexamers. Phi29 DNA polymerase reactions were carried out in 10 µL reaction volumes according to the following protocol: 1 µL 10x Phi29 polymerase buffer (Epicentre), 75 units Phi29 DNA polymerase (Epicentre), 75 ng template DNA, 1 µM Lomer or random hexamer primer and 0.4 mM dNTPs. Reaction mixtures were incubated at 95° C for 3 min without enzyme, after cooling on ice and addition of Phi29 DNA polymerase lomers and conventional hexamers were incubated at 25°C for 16h in presence or absence of human genomic DNA. The hexamer primed reactions yielded DNA even in absence of template whereas lomers only showed product formation in presence of template (figure 3).

**Table 1: Synthesized DNA sequences for lomer Experiments**

| **Name** | **SeqID** | **Sequence (5'->3')** |
|---|---|---|
| Lomer0 | 1 | aatgatacggcgacc |
| Lomer1 | 2 | aatgatDcggcgDccac |
| Lomer2 | 3 | aatgatDcggcgaDcac |
| Lomer3 | 4 | aatgataDggcgaDcac |
| Lomer4 | 5 | aatgdtacgDcgacDacc |
| Template0 | 6 | ttactatgccgctggtggctctagatgtgagaaagggatgtgctgcgagaaggctaga |
| Templatel | 7 | ttactaAgccgctggtggctctagatgtgagaaagggatgtgctgcgagaaggctaga |
| Template2 | 8 | ttactatgccgGtggtggctctagatgtgagaaagggatgtgctgcgagaaggctaga |
| Template3 | 9 | ttactaAgccgctggtggctctagatgtgagaaagggatgtgctgcgagaaggctaga |
| LomerHep | | NNNDNN*N*N |
| Random Hexamer | | NNNN*N*N |
| | | D: abasic spacer; *: phosphorothioate |

### Figure Legends

Figure 1: Schematic overview of lomer priming assays and resulting extension efficiencies.
Figure 2: Agarose gel electrophoresis of the priming assays for applied lomer primer/template combinations at 30 and 50 °C using Klenow fragment.
Figure 3: Comparision of whole genome amplification by random lomer primers and random hexamer primers.

## Claims

1. A method for isothermal amplification of a nucleic acid comprising the steps of:
a. combining in a reaction mixture one or more template nucleic acids, a nucleic acid polymerase, at least one polynucleotide comprising at least one backbone modification selected from the group comprising a d-spacer (abasic site), a short linker, an unlocked nucleic acid, a glycol nucleic acid, a triazole linker and an introduced additional negative charge as a primer, optionally one or more unmodified polynucleotides, a buffer, and deoxynucleotide triphosphates (dNTPs).
b. performing an amplification reaction at between 15 °C to 65 °C.

2. A method according to claim 1, wherein the at least one polynucleotide comprises at least two backbone modifications selected from the group comprising a d-spacer (abasic site), a short linker, an unlocked nucleic acid, a glycol nucleic acid, a triazole linker and an introduced additional negative charge.

3. A method according to any of the claims 1 or 2, wherein the amplification reaction is a whole genome amplification (WGA), a random primer whole genome amplification, a rolling circle amplification (RCA), loop-mediated isothermal amplification (LAMP), a helicase dependent amplification (HDA) or a multiple displacement amplification (MDA).

4. A method according to any of the claims 1 to 3, wherein the modified polynucleotide is used as a specific primer and allows specific binding to at least 6 bases.

5. A method according to any of the claims 1 to 3, wherein the modified polynucleotide is a random primer.

6. A method according to any of the claims 1 to 5, wherein the amplicon is further analyzed by sequencing and/ or wherein the amplicon is identified by hybridization with a labeled probe, by the accumulation of pyrophosphate, by quenching of 2-aminopurine or other labeled bases in the oligonucleotide primers, by DNA binding dyes, e.g. SybrGreen or ethidium bromide or by intercalating labels for abasic sites in duplex DNA.

7. A polynucleotide having a melting temperature of between 15 °C and 65 °C and comprising a combination of at least two backbone modifications selected from the group of a d-spacer (abasic site), a short linker, an unlocked nucleic acid, a glycol nucleic acid, a triazole linker and an additional negative charge.

8. A polynucleotide according to claim 7 with a length of less than 100 nucleotides.

9. A polynucleotide according to any of the claims 7 or 8, wherein the additional negative charge is a linker to the sugar side of the base, a ribose modification or a phosphate analogue.

10. A polynucleotide according to any of the claims 7 to 9, which allows a specific binding to at least 6 bases, preferably at least 15 bases, more preferably at least 20 bases of the target nucleic acid.

11. A polynucleotide according to any of the claims 7 to 10, which can be amplified by an enzyme with amplification activity selected from the group comprising Bst DNA polymerase, *Thermococcus species* 9°N-7 DNA polymerase, KlenTaq polymerase, Klenow polymerase and pol Y family polymerases.

12. A polynucleotide according to any of the claims 7 to 11, which carries further modifications.

13. Use of a polynucleotide according to any of the claims 7 to 12 as a primer for the isothermal amplification of a target nucleic acid.

14. Kit comprising at least one polynucleotide according to any of the claims 7 to 12, a nucleic acid polymerase and optionally a buffer and/or deoxynucleotide triphosphates.


SEQUENCE LISTING

<110>  Alacris Theranostics GmbH

<120>  Low Melting Temperature Primer

<130>  B106-0027EP1

<160>  9

<170>  PatentIn version 3.5

<210>  1
<211>  15
<212>  DNA
<213>  Artificial Sequence

<220>
<223>  artificial primer sequence without modified bases

<400>  1
aatgatacgg cgacc                                                        15


<210>  2
<211>  15
<212>  DNA
<213>  Artificial Sequence

<220>
<223>  artificial primer sequence with two modified bases (abasic sites)


<220>
<221>  misc_feature
<222>  (7)..(7)
<223>  abasic site at position 7

<220>
<221>  misc_feature
<222>  (13)..(13)
<223>  abasic site at position 13

<400>  2
aatgatcggc gccac                                                        15


<210>  3
<211>  15
<212>  DNA
<213>  Artificial Sequence

<220>
<223>  artificial primer sequence with two modified bases (abasic sites)


<220>
<221>  misc_feature
<222>  (7)..(7)
<223>  abasic site at position 7

<220>
<221>  misc_feature
<222>  (14)..(14)
<223>  abasic site at position 14

<400>  3
aatgatcggc gacac                                                        15


<210>  4
<211>  17
<212>  DNA
<213>  Artificial Sequence

<220>
<223>  artificial primer sequence with two modified bases (abasic sites)


<220>
<221>  misc_feature
<222>  (8)..(8)
<223>  abasic site at position 8

<220>
<221>  misc_feature
<222>  (14)..(14)
<223>  abasic site at position 14

<400>  4
aatgatangg cgancac                                                      17


<210>  5
<211>  15
<212>  DNA
<213>  Artificial Sequence

<220>
<223>  artificial primer sequence with three modified bases (abasic
       sites)


<220>
<221>  misc_feature
<222>  (5)..(5)
<223>  abasic site at position 5

<220>
<221>  misc_feature
<222>  (10)..(10)
<223>  abasic site at position 10

<220>
<221>  misc_feature
<222>  (15)..(15)
<223>  abasic site at position 15

<400>  5
aatgtacgcg acacc                                                        15


<210>  6
<211>  58
<212>  DNA
<213>  Artificial Sequence

<220>
<223>  artificial template sequence to test modified polynucleotides

<400>  6
ttactatgcc gctggtggct ctagatgtga gaaagggatg tgctgcgaga aggctaga        58


<210>  7
<211>  58
<212>  DNA
<213>  Artificial Sequence

<220>
<223>  artificial sequence to test modified polynucleotide primers

<400>  7
ttactaagcc gctggtggct ctagatgtga gaaagggatg tgctgcgaga aggctaga        58


<210>  8
<211>  58
<212>  DNA
<213>  Artificial Sequence

<220>
<223>  artificial sequence to test modified polynucleotide primers

<400>  8
ttactatgcc ggtggtggct ctagatgtga gaaagggatg tgctgcgaga aggctaga        58


<210>  9
<211>  58
<212>  DNA
<213>  Artificial Sequence

<220>
<223>  artificial sequence to test modified polynucleotide primers

<400>  9
ttactatgcc ggtggtggct ctagatgtga gaaagggatg tgctgcgaga aggctaga        58